# EUROPEAN PATENT APPLICATION

(11) **EP 4 759 920 A1**
(43) Date of publication of application: **17.06.2026**
(21) Application number: 24851898.7
(22) Date of filing: 08.08.2024
(51) Int. Cl.: C12N 1/16, C12P 7/6463

(54) **OIL-AND-FAT-PRODUCING YEAST MUTANT STRAIN THAT EXCESSIVELY ACCUMULATES OIL AND FAT**

(30) Priority: 08.08.2023 JP 2023129032
(71) Applicant: Teikyo University, Tokyo 173-8605 (JP)
(72) Inventor: TAKAYAMA, Yuko, Utsunomiya-shi, Tochigi 320-8551 (JP)
(74) Representative: Cohausz & Florack
(86) International application number: PCT/JP2024/028356
(87) International publication number: WO 2025/033480

(57) **Abstract**

Provided is an oleaginous yeast mutant strain with higher lipid accumulation than that of known genus *Lipomyces.* The mutant strain is a *Lipomyces starkeyi* strain with an accession number of NITE BP-03940.

## Description

### TECHNICAL FIELD

The present invention relates to an oleaginous yeast mutant strain that excessively accumulates lipids.

### BACKGROUND ART

Since genus *Lipomyces,* oleaginous yeasts, accumulate approximately 70% lipids per cell, and their lipid constituents are similar to those contained in palm oil, it is expected to be utilized not only as a biofuel, but also as an alternative oil for food and cosmetics, leading to rapid advances in research.

The inventor has developed cell biology research methods to elucidate the molecular mechanisms of lipid production in *Lipomyces.* In the process, we developed a medium for inducing lipid accumulation that can reduce the cost of the medium itself and can increase intracellular lipid accumulation (Patent literature 1).

However, the demand for lipid-production microorganisms and methods with high lipid production efficiency remains unchanged.

### CITATION LIST

### PATENT LITERATURE

[Patent literature 1] JP 2021-058103 A

### SUMMARY OF INVENTION

### TECHNICAL PROBLEM

An object of the present invention aims to provide an oleaginous yeast mutant strain with higher lipid accumulation than that of known genus *Lipomyces,* in particular, an oleaginous yeast mutant strain with even higher lipid accumulation under specific culture conditions.

### SOLUTION TO PROBLEM

The object can be solved by the following present inventions:
[1] A *Lipomyces starkeyi* strain with an accession number of NITE BP-03940.
[2] A method for inducing lipid accumulation by using the strain of [1].
[3] A method for inducing lipid accumulation, comprising:
   pre-culturing the strain of [1] until a stationary phase, and
   transferring the cells obtained by the pre-culture to a medium for main culture, and performing main culture.

### ADVANTAGEOUS EFFECTS OF INVENTION

According to the present invention, an oleaginous yeast mutant strain with higher lipid accumulation than that of known genus *Lipomyces* can be provided. Furthermore, by utilizing the mutant strain of the present invention, a more efficient method for inducing lipid accumulation can be provided.

### BRIEF DESCRIPTION OF THE DRAWINGS

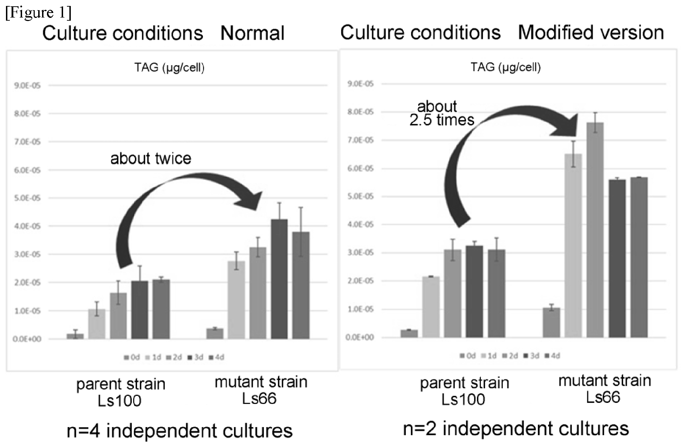

[Fig. 1] The graph on the left side of Figure 1 shows the amount of triacylglycerol (TAG) per cell (unit: µg/cell) when cultured under normal culture conditions, and the graph on the right side of Figure 1 shows the amount of triacylglycerol per cell when cultured under specific culture conditions. The bar graphs on the left side of each graph (from the left: 0 days, 1 day, 2 days, 3 days, 4 days) represent the results of the control, and the bar graphs on the right side (same as above) represent the results of the oleaginous yeast mutant strain of the present invention.

### DESCRIPTION OF EMBODIMENTS

### (Oleaginous yeast mutant strain of the present invention)

The oleaginous yeast mutant strain of the present invention is a *Lipomyces starkeyi* Ls66 strain (accession number: NITE BP-03940). The mutant strain of the present invention exhibits higher lipid accumulation than that of known genus *Lipomyces,* and can be used as an efficient lipid production microorganisms or oleaginous microorganisms.

Mycological characteristics of the Ls66 strain are as follows:
The strain is a budding yeast, and proliferates well in YPD medium (The medium composition will be described in the Examples below.). The strain is a diploid cell, and cannot induce spore formation in a "spore formation medium for natural yeast" and a "method of spore formation for natural yeast" disclosed in a patent application (Japanese Patent Application No. 2019-182882; JP 2021-058102 A) for which the inventor is the present inventor of the present application. The "spore formation medium for natural yeast" consists solely of water and agar, and the "method of spore formation for natural yeast" comprises the step of culturing the yeast on the agar medium consisting solely of water and agar.

### (Method for inducing lipid accumulation of the present invention)

The method for inducing lipid accumulation of the present invention can be carried out according to known methods for inducing lipid accumulation, except for the step of culturing using the oleaginous yeast mutant strain of the present invention.

The method for inducing lipid accumulation of the present invention may be carried out, for example, by pre-culturing the strain prior to the main culture, and by inoculating the prepared culture into an appropriate medium for inducing lipid accumulation to perform main culture. The main culture may be carried out at a temperature of, for example, 20 to 35°C, and for approximately 1 to 7 days under shaking conditions.

Furthermore, lipid accumulation may be carried out more efficiently by culturing the oleaginous yeast mutant strain of the present invention under specific culture conditions.

Lipid production may be carried out, for example, by performing the pre-culture in YPD medium, and inoculating the culture into N medium (The medium composition will be described in the Examples below.). Additionally, in a preferred embodiment of the present invention, lipid production may be further increased by performing the pre-culture in YPD medium until the stationary phase.

In the present invention, lipid accumulation in oleaginous yeast cells can be confirmed and quantified using known methods. Examples of such methods include a method in which after lipids are stained with a stain solution such as Oil Red, yeasts are photographed under a microscope and the lipids are visually confirmed; and a method in which a cell extract is prepared by dissolving or physically disrupting cell walls, and lipid components present in the extract are separated and qualified (for example, commercially available measurement kits (for example, Triglyceride E-Test Wako (Wako)).

### EXAMPLES

The present invention will now be further illustrated by, but is by no means limited to, the following Examples.

### <<Example 1: Isolation and identification of oleaginous yeast mutant strains>>

In this Example, we used a *Lipomyces starkeyi* Ls100 strain (deposited with NBRP), a derivative of a *Lipomyces starkeyi* NBRC1289 strain (obtained from NBRP (National BioResource Project)), which was a wild-type strain isolated from soil. After ultraviolet irradiation, screening was performed using triacylglycerol accumulation as an indicator. As described later, we successfully obtained an oleaginous yeast mutant strain (designated Ls66) exhibiting higher lipid accumulation than that of the NBRC1289 strain or the Ls100 strain even under normal culture conditions, and further exhibiting even higher lipid accumulation under specific culture conditions.

### << Example 2: Measurement of intracellular lipid accumulation in oleaginous yeast mutant strains>>

In this Example, the *Lipomyces starkeyi* Ls100 strain was used as a control, and the time course of intracellular lipid accumulation in the *Lipomyces starkeyi* Ls66 strain (accession number: NITE BP-03940), which is the oleaginous yeast mutant strain according to the present invention was measured. The experiments were conducted under normal culture conditions (four independent cultures; N=4) and under specific culture conditions in the present invention (two independent cultures; N=2).

### (Normal culture conditions)

Under normal culture conditions, cells were pre-cultured at 26°C in YPD medium (2% D-glucose, 1% yeast extract, 2% hipolypeptone), and cells in the logarithmic growth phase were harvested. After washing three times with sterile water, cells were transferred to N medium (0.3% sucrose, 0.025% KH₂PO₄, 0.066% MgSO₄·7H₂O, trace element solution (FeCl₃·6H₂O 1.7 mg/L, MnSO₄·6H₂O 0.51 mg/L, ZnSO₄·7H₂O 4.5 mg/L)) at a cell concentration of approximately 3 × 10⁶ cells/mL, and the culture was started at 26°C. Every other day, 5 × 10⁷ cells were harvested, and the amount of triacylglycerol was quantified.

### (Specific culture conditions in the present invention)

Under specific culture conditions in the present invention, cells were pre-cultured at 26°C in YPD medium until reaching a stationary state (3 × 10⁸ cells/mL), and then harvested. After washing three times with sterile water, cells were transferred to N medium at a cell concentration of approximately 4 × 10⁶ cells/mL, and the culture was started at 26°C. Every other day, 5 × 10⁷ cells were harvested, and the amount of triacylglycerol was quantified.

The results are shown in Figure 1. The graph on the left side of Figure 1 shows the amount of triacylglycerol (TAG) per cell (unit: µg/cell) under normal culture conditions, and the graph on the right side of Figure 1 shows the amount of triacylglycerol per cell under specific culture conditions. The bar graphs on the left side of each graph (from the left: 0 days (at the start of culture), 1 day, 2 days, 3 days, 4 days) represent the results of the control, and the bar graphs on the right side (same as above) represent the results of the oleaginous yeast mutant strain of the present invention.

Even under normal culture conditions (cells at 0 days from culture initiation were in the logarithmic growth phase), the lipid accumulation was approximately twice that of the control Ls100 strain (Figure 1, left graph).

Furthermore, under specific culture conditions (cells at 0 days from culture initiation were in the stationary phase), the lipid accumulation was approximately 2.5 times higher than that of the control Ls100 strain, indicating an additional increase in lipid accumulation (Figure 1, right graph).

It was confirmed that the oleaginous yeast mutant strain of the present invention could increase lipid production efficiency compared to the known Ls100 strain simply by replacing a novel strain, and could further increase lipid production efficiency by modifying the culture conditions.

### INDUSTRIAL APPLICABILITY

The oleaginous yeast mutant strain of the present invention can be utilized in the field of lipid production using yeasts.

### REFERENCE TO DEPOSITED BIOLOGICAL MATERIAL

*Lipomyces starkeyi* Ls66 was deposited internationally at the NITE Patent Microorganisms Depositary (NPMD), National Institute of Technology and Evaluation (Address: #122, 2-5-8 Kazusakamatari, Kisarazu-shi, Chiba 292-0818, Japan) on July 11, 2023 under accession number NITE BP-03940.

## Claims

1. A *Lipomyces starkeyi* strain with an accession number of NITE BP-03940.

2. A method for inducing lipid accumulation by using the strain according to claim 1.

3. A method for inducing lipid accumulation, comprising:
pre-culturing the strain according to claim 1 until a stationary phase, and transferring the cells obtained by the pre-culture to a medium for main culture, and performing main culture.
